(19) Europäisches Patentamt
European Patent Office
Office européen des brevets

(11) EP 1 223 225 A1

(12) EUROPEAN PATENT APPLICATION

(43) Date of publication:
**17.07.2002 Bulletin 2002/29**

(51) Int Cl.[7]: **C12Q 1/68**

(21) Application number: **01200057.6**

(22) Date of filing: **10.01.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **STICHTING NEDERLANDS INSTITUUT VOOR ZUIVELONDERZOEK**
**6710 BA Ede (NL)**

(72) Inventor: **Herrewegh, Arnold Aloysius Philomena Maria**
**3607 KE Maarssen (NL)**

(74) Representative: **Jorritsma, Ruurd et al**
**Nederlandsch Octrooibureau**
**Scheveningseweg 82**
**P.O. Box 29720**
**2502 LS Den Haag (NL)**

# (54) Detection of mycobacterium avium ssp paratuberculosis

(57) The present invention relates to methods for the PCR-based detection of nucleic acids from *Mycobacterium avium* ssp. *paratuberculosis (Mycobacterium paratuberculosis),* in biological samples such as milk. The oligonucleotide primers to be used in the method of the invention are designed so as to improve the specificity of detection of *Mycobacterium avium* ssp. *paratuberculosis* by avoiding detection of related environmental *Mycobacteria.* The sensitivity of detection is improved by using an optimized procedure for extraction of DNA from milk and by using a nested PCR method.



Printed by Jouve, 75001 PARIS (FR)

## Description

### Field of the Invention

The present invention relates to the PCR-based detection of nucleic acids from *Mycobacterium avium* ssp. *paratuberculosis (Mycobacterium paratuberculosis)*, in biological samples such as milk.

### Background of the Invention

Paratuberculosis, or Johne's disease, is a chronic enteritis of ruminants which is caused by *Mycobacterium paratuberculosis*. Prevalance in cattle worldwide ranges form 0 to 40% and the disease also affects other ruminants such as sheep and goats. More recently, *M. paratuberculosis* has also been implicated in Crohn's disease (regional ileitis) of humans, which has further increased the need to control the spread of *M. paratuberculosis*.

Control of Johne's disease has been seriously hampered by inadequate and time consuming diagnostic procedures. *M. paratuberculosis* is an intracellular pathogen which replicates in macrophages in the lamina propria of the intestines and is shed into the feces during disease. Initially, diagnosis involved culture *of M paratuberculosis* from feces. However, *M paratuberculosis* requires complex culture conditions for growth *in vitro* and is extremely slow growing. Primary isolation of the organism from feces would usually take 6 - 12 weeks. In addition, infected animals may only intermittently shed the microorganism, as a result of which false negatives will occur. Similarly, diagnosis on the basis of immunological techniques has been hampered by false negatives, and also by false positive results due to prior immunological exposure or cross-reactivity of the test to related environmental mycobacteria.

More recently, DNA-based techniques have been developed for the detection *of M. paratuberculosis*. EP 0 288 306 discloses DNA probes for the detection of mycobacteria based on DNA sequences derived from the mycobacterial insertion sequence IS *900* (also referred to as ISM1). IS *900* was identified in a clone derived from genomic library prepared from a human Crohn's disease isolate of *M. paratuberculosis*. The IS *900* sequence is found to be repeated 15 to 20 times in the genome of *M. paratuberculosis*. EP 0 288 306 further discloses the DNA sequence of IS *900,* methods for identifying and differentiating mycobacteria using IS 900-based probes in DNA fingerprinting and suggests using IS *900* sequences in methods for detecting mycobacteria using polymerase chain reaction (PCR). However, EP 0 288 306 does not disclose any IS *900*-based primers for this purpose.

Vary et al. (1990; J. Clin. Microbiol. 28: 933-937) disclose direct and specific detection of *M. paratuberculosis* in feces from cattle with Johne's disease using primers based on IS *900* sequences in a PCR. The PCR test has a sensitivity at least equal to that routinely obtained in the conventional culture techniques.

Millar et al. (1995; Anal. Biochem. 226: 325-330) combine a solid phase hybridization capture system for the enrichment of *M. paratuberculosis* target sequences with IS *900* based PCR to increase the sensitivity of the reaction to 10-100 copies of *M. paratuberculosis* per gram of feces.

With the implication of *M. paratuberculosis* in Crohn's disease in human the need for tests for detection *of M paratuberculosis* in cow's milk for human consumption has arisen. Millar et al. (1996; Appl. Environ. Microbiol. 62: 3446-3452) disclose an IS *900*-based PCR procedure for detection of *M. paratuberculosis* in both raw and pasteurized cow's milk. The sensitivity of the procedure is estimated to lie in the range of 200 - 300 *M. paratuberculosis* bacilli per ml of milk.

A problem with the IS *900*-based PCR detection of *M. paratuberculosis* is that IS *900* is part of a family of DNA insertion sequences in the actinomycetes. Non-pathogenic environmental mycobacteria such as *M. avium* ssp. *avium* are known to contain insertion sequences IS *901* and IS *1613* both of which have a high sequence homology with the IS900 sequence of *M. paratuberculosis.* Consequently, PCR primers may not be able to distinguish between these related insertion sequences, particularly if their 3'-end is homologous to the non-IS *900* insertion sequences. This will compromise the selectivity of IS *900*-based PCR detection of *M. paratuberculosis* and may lead to false positive results.

Thus, there is still a need for an IS *900*-based PCR test for the detection of *M. paratuberculosis* with improved sensitivity and improved specificity.

### Description of the Invention

### Definitions

The term "oligonucleotide" as used herein includes linear oligomers of natural or modified monomers or linkages, including deoxyribonucleosides, ribonucleosides, .alpha.-anomeric forms thereof, polyamide nucleic acids, and the like, capable of specifically binding to a target polynucleotide by way of a regular pattern of monomer-to-monomer interactions (e.g. nucleoside-to-nucleoside), such as Watson-Crick type of base pairing, Hoogsteen or reverse Hoogsteen types of base pairing, or the like. Usually monomers are linked by phosphodiester bonds or analogs thereof to form oligonucleotides ranging in size from a few monomeric units, e.g. 3-4, to several hundreds of monomeric units, although usually no more than 100 monomeric units. Whenever an oligonucleotide is represented by a sequence of letters, such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, unless otherwise noted. Analogs of phosphodiester linkages include phosphorothioate, phosphorodithioate, phosphoroselenoate, phosphorodiselenoate, phosphoroanilothioate, phosphoranilidate, phosphoramidate and the like.

The term "primer" as used herein refers to an oligonucleotide whether occurring naturally as in a purified restriction digest or produced synthetically by chemical and/or enzymatic synthesis, which is capable of acting as a point of initiation of synthesis when placed under conditions in which synthesis of a primer extension product which is complementary to a nucleic acid strand is induced, i.e., in the presence of nucleotide and an agent for polymerization such as DNA polymerase and at a suitable temperature and pH. The primer is preferably single-stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerization. The exact lengths of the primers will depend on many factors, including temperature and source of primers. Oligonucleotide primers will typically contains 15-30 or more nucleotides, although in the context of the present invention primers will preferably contain 18-24 nucleotides.

Unless specified otherwise, the conventional notation used herein portrays polynucleotides as follows: the left-hand end of single-stranded polynucleotide sequences is the 5' end; the left-hand direction of double-stranded polynucleotide sequences is referred to as the 5' direction. A location in a polynucleotide is referred to as "upstream" of a reference point in that polynucleotide if that location is closer to the 5'-end of the polynucleotide with respect to the reference point. A location in a polynucleotide is referred to as "downstream" of a reference point in that polynucleotide if that location is closer to the 3'-end of the polynucleotide with respect to the reference point. "Polymerase Chain Reaction" or "PCR" is a rapid procedure for in vitro enzymatic amplification of a specific DNA segment. The DNA to be amplified is denatured by heating the sample. In the presence of DNA polymerase and excess deoxynucleotide triphosphates, oligonucleotides that hybridize specifically to the target sequence prime new DNA synthesis. One round of synthesis results in new strands of indeterminate length which, like the parental strands, can hybridize to the primers upon denaturation and annealing. The second cycle of denaturation, annealing and synthesis produces two single-stranded products that together compose a discrete double-stranded product, exactly the length between the primer ends. This discrete product accumulates exponentially with each successive round of amplification. Over the course of about 20 to 45 cycles, many million-fold amplification of the discrete fragment can be achieved. PCR protocols are well known in the art, and are described in standard laboratory textbooks, e.g. Ausubel et al., "Current Protocols in Molecular Biology", John Wiley & Sons, Inc. (1995).

The melting temperature (indicated as "$T_m$") of a DNA molecule, herein usually an oligonucleotide, is understood to mean the temperature at which the transition of double-stranded DNA to single-stranded DNA is halfway complete during thermal denaturation. The $T_m$ of an oligonucleotide of known sequence may be calculated using the Nearest Neighbor Algorithm for $T_m$, formula:

$$T_m = \frac{\Delta H^\circ}{\Delta S^\circ + (R \times \ln(C_T))} - 273.15$$

wherein $\Delta H^\circ$ is the enthalpy and $\Delta S^\circ$ is the entropy for helix formation, R is the molar gas constant (1.987 cal/K·mol), and $C_T$ is the total primer concentration (Beslauer, K.J., Frank, R., Blocker, H. and Marky, L.A. 1986. Proc.Natl.Acad. Sci. 83:3746-3750.

*"Mycobacterium paratuberculosis"* has recently been (re)classified as a subspecies of *Mycobacterium avium* and consequently has been renamed as *Mycobacterium avium* ssp. *paratuberculosis* (Thorel, MF, Krichevsky, M., Levy-Frebault V.V. (1990) Int. J. Syst. Bacteriol. 40:254-260). It is, therefore, understood that the term "*M avium* ssp. *paratuberculosis"* as used herein, equally refers to all bacteria (previously) referred to as *M. paratuberculosis*.

In a first aspect the invention relates to oligonucleotides corresponding in sequence to a part of SEQ ID NO. 1 (i.e. IS *900),* or to its complementary sequence. The oligonucleotides may be used as primers in PCR-based detection of *M. avium* ssp. *paratuberculosis*. For improved specificity and sensitivity of the PCR detection, the oligonucleotides of the invention further comply with one or more of the following criteria: (a) the length of the oligonucleotides is 14 to 30 nucleotides, preferably 18 to 24 nucleotides; (b) the G/C content of the oligonucleotides is 37% to 70%; (c) the melting temperature of the oligonucleotides ranges from 55 to 65 °C; (d) the oligonucleotides do not contain more than 5 contiguously repeated bases of the same type, e.g. the oligonucleotides do not contain more than 5 consecutive adenosines; (e) the oligonucleotides do not contain more than 7 contiguous residues selected from the group consisting of G and C residues; (f) the total number of G and C residues in the oligonucleotides ranges from 9 to 13. Thus, e.g. an oligonucleotide of 14 nucleotides will comprise no more than 9 G and/or C residues (limited by the 70% G/C) and an oligonucleotide of 30 nucleotides will comprise no more than 13 G and/or C residues (limited by the number 13 which in this case corresponds to 43% G/C); (g) the oligonucleotides have at least two mismatches with SEQ ID NO. 2 (i.e. IS *901)* and with SEQ ID NO. 3 (i.e. IS *1631*), of which at least one mismatch with each SEQ ID NO. 2 and with SEQ ID NO. 3 occurs in the three most extreme nucleotides at the 3'-end of the oligonucleotides. Preferably, the oligonucleotides have at least four mismatches with SEQ ID NO. 2 and with SEQ ID NO. 3, of which at least two mismatches with each SEQ ID NO. 2 and SEQ ID NO. 3 occur in the three most extreme nucleotides at its 3'-end, of which at least one mismatch with either SEQ ID NO. 2 or SEQ ID NO occurs at the most extreme nucleotide at its 3'-end. More preferably, the oligonucleotides have at least five, six, seven or eight mismatches with SEQ ID NO. 2 and with SEQ ID NO. 3, of which at least two mismatches, with both of each SEQ ID NO. 2 and SEQ ID NO. 3, occur in the three most extreme nucleotides at its 3'-end, of which at least one mismatch with either SEQ ID NO. 2 or SEQ ID NO. 3 occurs at the most extreme nucleotide at its 3'-end. In a preferred embodiment of the invention, the oligonucleotide of the invention has a nucleotide sequence selected from the group consisting of SEQ ID NO. 4 - SEQ ID NO. 38. Figure 1 presents an alignment of the DNA sequences of IS *900* (SEQ ID NO. 1), IS *901* (SEQ ID NO.2) and IS *1631* (SEQ ID NO.3). Using this sequence comparison the skilled person will be capable of designing other oligonucleotides according to the invention.

A further aspect of the invention relates to a method for detection of *M. avium* ssp. *paratuberculosis* in a sample by means of amplification and detection of *M. avium* ssp. *paratuberculosis-specific* nucleic acids by PCR. The methods is applicable to any kind of sample but will typically be performed on samples obtained from a human or animal subject, such as e.g. blood, feces, urine, saliva, tissue, or milk. The sample may be obtained from more than one subject, e.g. in the dairy industry sample may be taken from batches of pooled milk obtained from many different animals. The method will usually comprise the step of preparing the sample for PCR, which may involve extraction of template DNA from the sample. A variety of methods for sample preparation for PCR are known per se to the skilled person and are described in Innis et al., "PCR protocols" Academic Press (1990) and in Ausubel et al., "Current Protocols in Molecular Biology", John Wiley & Sons, Inc. (1995). A very simple method for PCR sample preparation consists of no more than mixing or suspending a sample in an appropriate amount of (demineralized) water and heating the sample for at least 1 minute, preferably 3 to 5 minutes, at a temperature of at least 90°C, preferably at least 95°C or 100°C, and, optionally, centrifuging the sample to remove any insoluble debris (e.g. for 3 minutes in a microcentrifuge). The Examples herein provide a method for PCR sample preparation which is optimized for highly sensitive PCR-based detection of DNA in milk, e.g. to be applied for PCR-based detection of the presence of (pathogenic) micro-organisms, such as *M. avium* ssp. *paratuberculosis*, in milk.

In the method for detection of *M. avium* ssp. *paratuberculosis* in a sample a PCR is performed on the sample, wherein a first and a second oligonucleotide of the invention are used as PCR primers, of which the first oligonucleotide is of the same orientation as SEQ ID NO. 1 (sense) and is located upstream of the second oligonucleotide with respect to SEQ ID NO. 1 and of which the second oligonucleotide is complementary to SEQ ID NO. 1 (antisense). Preferably, the first and second oligonucleotides are chosen such that the resulting PCR product is less than 1000 bp, preferably less than 750 bp or 500 bp but preferably more than 50 bp, preferably more than 75 bp. Typical combinations of first and second oligonucleotides are e.g. a first oligonucleotide selected from any one of SEQ ID NO.4, SEQ ID NO. 5, SEQ ID NO. 6, SEQ ID NO. 7, SEQ ID NO. 8, SEQ ID NO. 9, SEQ ID NO. 10, and a second oligonucleotide selected from any one of SEQ ID NO. 24, SEQ ID NO. 25, SEQ ID NO. 26, SEQ ID NO. 27, SEQ ID NO. 28, SEQ ID NO. 29, SEQ ID NO. 30.

The PCR conditions are generally well known in the art (see e.g. Innis et al., "PCR protocols" Academic Press (1990) and in Ausubel et al., "Current Protocols in Molecular Biology", John Wiley & Sons, Inc. (1995).). Usually at least 25 cycles are performed, preferably at least 30, 35 or 40 cycles but in general no more than 45 cycles are required. As a typical but non-limiting example, one cycle may consist of 25 seconds denaturation at 94°C, followed by 20 seconds annealing at a temperature which is 5°C below the Tm of the primer with the lowest Tm, followed by 50 seconds of polymerization (primer extension) at 72°C.

Buffer conditions, i.e. type of buffer, pH, triphosphate nucleotide concentrations and mono- and di-valent cation concentration are well known in the art from *inter alia* the above referenced textbooks. Similarly, polymerases suitable for PCR are well known in the art and include the *Taq* polymerase as used herein as well as other polymerases from both meso- and thermo-philic organisms such as DNA polymerase I - Klenow fragment, Vent™ and Deep-Vent™ DNA polymerases, and *Tfl* DNA polymerase.

In a preferred aspect of the invention a so-called "nested PCR" is applied for the detection of *M. avium* ssp. *paratuberculosis* in order to further improve both the sensitivity and the specificity of the PCR. This aspect of the invention relates to a method for PCR-based detection of *M. avium* ssp. *paratuberculosis*, wherein the PCR product obtained in the first stage PCR with the first and second oligonucleotides is used as template in a second stage PCR, in which a third and a fourth oligonucleotide are used as primers. The third oligonucleotide is of the same orientation as SEQ ID NO. 1 and is located downstream of the first oligonucleotide but upstream of the second oligonucleotide and upstream of the fourth oligonucleotide with respect to SEQ ID NO. 1. The fourth oligonucleotide is complementary to SEQ ID NO. 1 and is located upstream of the second oligonucleotide with respect to SEQ ID NO.1. Typical combinations of first, second, third and fourth oligonucleotides are e.g. a first oligonucleotide selected from any one of SEQ ID NO. 4, SEQ ID NO. 6, or SEQ ID NO. 7, a second oligonucleotide selected from any one of SEQ ID NO. 24, SEQ ID NO. 28, or SEQ ID NO. 29, a third oligonucleotide selected from any one of SEQ ID NO. 8, SEQ ID NO. 9, SEQ ID NO. 10, and a fourth oligonucleotide selected from any one of SEQ ID NO. 25, SEQ ID NO. 26, or SEQ ID NO. 27. Table 1 provides an overview of oligonucleotides SEQ ID NO. 4- SEQ ID NO. 38, including the position and orientation of the oligonucleotides with respect to SEQ ID NO. 1, allowing the skilled person to select other combinations of four oligonucleotides suitable for nested PCR detection of *M. avium* ssp. *paratuberculosis*. Similarly, based on the DNA sequence comparison of Figure 1, the skilled person may design other oligonucleotides according to the invention which in combination are suitable for nested PCR detection of *M. avium* ssp. *paratuberculosis*.

Another aspect of the invention relates to the detection and/or identification of the amplified DNA fragment, if any, in the first- and second-stage PCRs. The amplified product may be directly or indirectly detected by incorporation of an appropriate visualizing label, as for example, a radioactive, calorimetric, fluorometric (ethidium bromide) or luminescent signal, or the like. Identification of the amplification products, as being derived from the *M. avium* ssp. *paratuberculosis* IS *900* insertion sequence (SEQ ID NO. 1), may be accomplished by any one of several methods known in the art to identify amplified nucleotide sequences. These methods include, but are not limited to, determination of size, restriction enzyme digestion pattern, subsequent cloning of amplification products, Southern blot hybridization with an oligonucleotide probe internal to the nucleotide sequence being amplified, or DNA sequencing. The amplified DNA products are optionally separated from the reaction mixture and then analyzed. The amplified gene sequences may be visualized, for example, by separating the gene sequences from undesirable side-products and unreacted reagents by electrophoresis in an agarose or polyacrylamide gel, by HPLC separation in an ion exchange column or size exclusion column, by Capilary Electrophoresis as described by Kuypers et al. (1994) J. Chromatogr. B, 660:271, or by a Enzyme Linked Oligonucleotide Sorbent Assay (ELOSA technique) as described by Luneberg et al.(1993) J. Clin. Microbiol. 31:1088 and Katz et al.(1993) Am. J. Vet. Res., 54:2021, and other like techniques known and used in the art. The methods of the invention for detection of *M. avium* ssp. *paratuberculosis* are preferably used to detect the presence or absence of this bacterium in milk samples, of which preferably sample of milk intended for human (or animal) consumption such as bovine, ovine, caprine, equine or human milk or collostrum.

The present invention also relates to of "kits" containing elements for detecting *M. avium* ssp. *paratuberculosis*. Such a kit may comprise a carrier to receive therein one or more containers, such as tubes or vials. Unlabeled or detectable labeled oligonucleotides of the invention, e.g. to be used as primers, may be contained in one or more of the containers. The oligonucleotides may be present in lyophilized form, or in an appropriate buffer. One or more enzymes or reagents for use in PCR reactions may be contained in one or more of the containers. The enzymes or reagents may be present alone or in admixture, and in lyophilised form or in appropriate buffers. The kit may also contain any other component necessary for carrying out the present invention, such as buffers, agents for extraction of DNA (e.g. from milk), enzymes, pipettes, plates, nucleic acids, nucleoside triphosphates, filter paper, gel materials, transfer materials, and autoradiography supplies. Such other components for the kits of the invention are known per se. Preferably a kit comprises at least two oligonucleotides according to the invention, of which a first oligonucleotide is of the same orientation as SEQ ID NO. 1 and is located upstream from a second oligonucleotide with respect to SEQ ID NO. 1 and wherein the second oligonucleotide is complementary to SEQ ID NO. 1. A more preferred kit further comprises a third and a fourth oligonucleotide according to the invention, of which the third oligonucleotide is of the same orientation as SEQ ID NO. 1 and is located downstream of the first oligonucleotide, upstream of the second oligonucleotide and upstream of the fourth oligonucleotide with respect to SEQ ID NO. 1, and of which the fourth oligonucleotide is complementary to SEQ ID NO. 1.

The following Examples serve to illustrate the invention and should by no means be construed so as to limit the scope of the invention.

## Examples

### Example I: Procedure for the extraction of nucleic acids from milk

To 25 ml of raw milk was added 5 ml 1-propanol, 5 ml 25% ammonia, 5 ml petroleum-ether, 5 ml diethylether and $10^6$ colony forming units of Lactobacillus plantarum (or any other non-sporeforming Gram-positive bacterium species). The milk with additions were thoroughly mixed and centrifuge for 20 min at 4500 rpm in a table centrifuge (Lactobacilli are added to act as a carrier for the minute amounts of Mycobacteria present in milk). Subsequently, the fatty upperlayer and the supernatant are removed, the pellet is resuspended in 1 ml bi-destilled water and transferred to a 1.5 ml reaction tube and centrifuged for 5 min at 3.000 rpm in a microcentrifuge. The supernatant is removed and 300 µl of a 6% Chelex 100 suspension in water is added and incubated for 30 min at 56 °C. 300 µl of GTC-buffer (5 M guanidinium thiocyanate ($CH_5N_3CHNS$) in 100 mM Tris/HCl pH 8.0) is added and heated for 15 min at 100 °C, followed by freezing at -20 °C, followed by another heating at 100 °C for 10 min. After centrifugation for 5 min at 12000 rpm in a microcentrifuge, 500 µl supernatant is transferred to a new reaction tube and 500 µl isopropanol is added followed by an incubate of 2 hours at -20 °C and subsequently centrifugation at 4 °C for 30 min at 14.000 rpm in a microcentrifuge. The supernatant is removed and the DNA pellet is washed in 70% ethanol, dried and dissolved in 20 µl $H_2O$. This DNA solution is used as a template for PCR amplification.

Example II: Nested PCR for the detection of *M. avium* ssp. *paratuberculosis* in milk

For nested-PCR amplification of *M. avium* ssp. *paratuberculosis*, the four primers IS900-01 (SEQ ID NO: 4 = CTTACCTTTCTTGAAGGGTGTTC), IS900-02 (SEQ ID NO 5 = GTATGGCTTTCATGTGGTTGCT), IS900-03 (SEQ ID NO: 25 = TAACCGTCATTGTCCAGATCAAC) and IS900-04 (SEQ ID NO: 24 = GTCGTTAATAACCATGCAGTAATG) were used.

First-PCR: to a 0.5 ml PCR tube was added, 5 µl 10x PCR buffer (100 mM Tris-HCl, 500 mM KCl, pH 9.0), 200 nM primer IS900-01, 200 nM primer IS900-04, 200 µM dATP, 200 µM dCTP, 200 µM dGTP, 200 µM dTTP, 2.5 mM $MgCl_2$, 5 µl DNA template, 1.5 units Taq polymerase, $H_2O$ to 50 µl. First-PCR programme: 3 min preincubation at 90 °C followed by 40 cycli of 25 sec at 94 °C, 20 sec at 60 °C, 50 sec at 72 °C. The reaction mixture of the first-PCR (which reveals a product of 302 basepairs) was used as template in the nested-PCR.

Second (nested)-PCR: to a 0.5 ml PCR tube was add, 5 µl 10x PCR buffer (100 mM Tris-HCl, 500 mM KCl, pH 9.0), 200 nM primer IS900-02, 200 nM primer IS900-03, 200 µM dATP, 200 µM dCTP, 200 µM dGTP, 200 µM dTTP, 1.5 mM $MgCl_2$, 5 µl first PCR template, 1.5 units Taq polymerase, $H_2O$ to 50 µl. Nested-PCR programme: 40 cycli of 25 sec at 94 °C, 20 sec at 60 °C, 50 sec. at 72 °C. Positive PCR samples reveal a band of 159 basepairs.

Example III: Sensitivity of the nested PCR assay

To determine the sensitivity of the nested PCR assay, 5 µl of plasmid pMD2 solution in water, containing 1, 10, 100, 1000, 10.000, and 100.000 copies of pMD2, respectively, was used as a template in the first PCR, followed by the second PCR. Plasmid pMD2 contains the 335 most 5' basepairs of IS900 cloned into pUC21. 10 µl amplified PCR product was run on a 2% agarose gel. With the first PCR, as little as 100 copies of pMD2 could be detected, corresponding to 100 copies of IS900. With the nested PCR, as little as 1 copy of pMD2 could be detected, corresponding to 1 copy of IS900.

Example IV: Detection of M. avium ssp. paratuberculosis in milk from infected cows.

The efficacy of the nested PCR to detect *M. avium* ssp. *paratuberculosis* in milk was investigated. DNA was extracted from 25 ml of raw milk obtained from nine cows having a *M avium* ssp. *paratuberculosis* infection and from ten cows free of *M. avium* ssp. *paratuberculosis*. The purified DNA was used as template in the nested PCR assay with primers IS900-01, IS900-02, IS900-03 and IS900-04. The PCR product of the second PCR was analyzed on a 2% agarose gel. The nine milk samples obtained from the cows with *M. avium* ssp. *paratuberculosis* of 159 bp. The 10 milk samples from uninfected cows revealed no amplification product..

Table 1.

| *M. paratuberculosis* primers and their position and orientation with respect to IS900 | | | | | | | | |
|---|---|---|---|---|---|---|---|---|
| Sequence | SEQ ID NO. | Length | A | C | G | T | Position | Orientation |
| IS900 | 1 | 1451 | 252 | 498 | 453 | 248 | 1 - 1451 | SENSE |
| P-IS01 | 4 | 23 | 3 | 5 | 5 | 10 | 3 - 25 | SENSE |
| P-IS02 | 5 | 22 | 2 | 3 | 7 | 10 | 66 - 87 | SENSE |
| P-IS03 | 25 | 23 | 7 | 7 | 3 | 6 | 202 - 224 | ANTI-SENSE |
| P-IS04 | 24 | 24 | 7 | 5 | 4 | 8 | 281 - 304 | ANTI-SENSE |
| P-IS05 | 6 | 22 | 3 | 4 | 7 | 8 | 6 - 27 | SENSE |
| P-IS06 | 7 | 22 | 2 | 4 | 8 | 8 | 7 - 28 | SENSE |
| P-IS07 | 8 | 23 | 1 | 3 | 8 | 11 | 70 - 92 | SENSE |
| P-IS08 | 9 | 23 | 1 | 3 | 8 | 11 | 71 - 93 | SENSE |
| P-IS09 | 10 | 23 | 1 | 3 | 8 | 11 | 72 - 94 | SENSE |

Table 1. (continued)

| *M. paratuberculosis* primers and their position and orientation with respect to IS900 | | | | | | | | |
|---|---|---|---|---|---|---|---|---|
| Sequence | SEQ ID NO. | Length | A | C | G | T | Position | Orientation |
| P-IS10 | 11 | 21 | 1 | 4 | 8 | 8 | 190 - 210 | SENSE |
| P-IS11 | 12 | 21 | 1 | 4 | 8 | 8 | 191 - 211 | SENSE |
| P-IS12 | 13 | 24 | 6 | 4 | 7 | 7 | 203 - 226 | SENSE |
| P-IS13 | 14 | 21 | 4 | 4 | 8 | 5 | 228 - 248 | SENSE |
| P-IS14 | 15 | 21 | 3 | 5 | 7 | 6 | 230 - 250 | SENSE |
| P-IS15 | 16 | 22 | 7 | 4 | 6 | 5 | 290 - 311 | SENSE |
| P-IS17 | 17 | 22 | 6 | 5 | 7 | 4 | 292 - 313 | SENSE |
| P-IS18 | 18 | 20 | 2 | 4 | 8 | 6 | 349 - 368 | SENSE |
| P-IS19 | 19 | 20 | 2 | 4 | 8 | 6 | 350 - 369 | SENSE |
| P-IS20 | 20 | 18 | 4 | 7 | 5 | 2 | 409 - 426 | SENSE |
| P-IS21 | 21 | 19 | 5 | 7 | 5 | 2 | 544 - 562 | SENSE |
| P-IS22 | 22 | 20 | 4 | 7 | 6 | 3 | 629 - 648 | SENSE |
| P-IS23 | 23 | 20 | 4 | 8 | 4 | 4 | 638 - 657 | SENSE |
| P-IS24 | 26 | 22 | 6 | 4 | 8 | 4 | 208 229 | ANTI-SENSE |
| P-IS25 | 27 | 22 | 6 | 3 | 9 | 4 | 209 - 230 | ANTI-SENSE |
| P-IS26 | 28 | 24 | 7 | 4 | 5 | 8 | 283 - 306 | ANTI-SENSE |
| P-IS27 | 29 | 20 | 6 | 6 | 6 | 2 | 298 - 317 | ANTI-SENSE |
| P-IS28 | 30 | 20 | 2 | 4 | 8 | 6 | 349 - 368 | ANTI-SENSE |
| P-IS29 | 31 | 19 | 2 | 3 | 9 | 5 | 361 - 379 | ANTI-SENSE |
| P-IS30 | 32 | 21 | 3 | 5 | 6 | 7 | 468 - 488 | ANTI-SENSE |
| P-IS31 | 33 | 18 | 4 | 7 | 4 | 3 | 560 - 577 | ANTI-SENSE |
| P-IS32 | 34 | 20 | 4 | 6 | 6 | 4 | 616 - 635 | ANTI-SENSE |
| P-IS33 | 35 | 20 | 3 | 6 | 6 | 5 | 647 - 666 | ANTI-SENSE |
| P-IS34 | 36 | 22 | 6 | 9 | 3 | 4 | 778 - 799 | ANTI-SENSE |
| P-IS35 | 37 | 18 | 2 | 6 | 7 | 3 | 819 - 836 | ANTI-SENSE |
| P-IS36 | 38 | 18 | 3 | 8 | 5 | 2 | 862 - 879 | ANTI-SENSE |

## Claims

1. An oligonucleotide corresponding in sequence to a part of SEQ ID NO. 1 or to its complementary sequence, wherein the oligonucleotide has:

    a) a length of 18 to 24 nucleotides;
    b) a G/C content of 37% to 70%
    c) a melting temperature of 55 - 65 °C;
    d) no more than 5 contiguously repeated bases;
    e) no more than 7 contiguous G and/or C residues;
    f) a total number of G and C residues of 9 to 13; and
    g) at least two mismatches with SEQ ID NO. 2 and with SEQ ID NO. 3, of which at least one mismatch with each SEQ ID NO. 2 and SEQ ID NO. 3 occurs in the three most extreme nucleotides at its 3'-end.

2. An oligonucleotide according to claim 1, wherein the oligonucleotide has at least four mismatches with SEQ ID NO. 2 and with SEQ ID NO. 3, of which at least two mismatches with each SEQ ID NO. 2 and SEQ ID NO. 3 occur in the three most extreme nucleotides at its 3'-end, of which at least one mismatch with either SEQ ID NO. 2 or SEQ ID NO. 3 occurs at the most extreme nucleotide at its 3'-end.

3. An oligonucleotide according to claims 1 or 2, wherein the oligonucleotide has a nucleotide sequence selected from the group consisting of SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38

4. A method for the detection of *M.avium* ssp. *paratuberculosis* in a sample, wherein the method comprises the step performing a PCR on the sample, wherein a first and a second oligonucleotide according to any one of claims 1 to 3 are used as PCR primers, of which the first oligonucleotide is of the same orientation as SEQ ID NO. 1 and is located upstream of the second oligonucleotide with respect to SEQ ID NO. 1 and of which the second oligonucleotide is complementary to SEQ ID NO. 1.

5. A method according to claim 4, wherein the method further comprises the step of performing a second PCR wherein a reaction product obtained in claim 4 is used as template and wherein a third and a fourth oligonucleotide according to any one of claims 1 to 3 are used as PCR primers, of which the third oligonucleotide is of the same orientation as SEQ ID NO. 1 and is located downstream of the first oligonucleotide, upstream of the second oligonucleotide and upstream of the fourth oligonucleotide with respect to SEQ ID NO. 1, and of which the fourth oligonucleotide is complementary to SEQ ID NO. 1 and is located upstream of the second oligonucleotide with respect to SEQ ID NO. 1.

6. A method according to claims 4 or 5, wherein the method further comprises the step of detecting the presence or absence of an amplified DNA fragment.

7. A method according to any one of claims 4 to 6, wherein the sample is milk.

8. A method according to any one of claims 4 to 7, wherein the sample is milk from a mammal selected from the group consisting of human, bovine, ovine, caprine, equine.

9. A method according to any one of claims 7 to 8, wherein the template DNA is extracted from the milk prior to amplification.

10. A kit for use in the methods of any one of claims 4 to 9, wherein the kit comprises at least two oligonucleotides according to any one of claim 1 to 3, of which a first oligonucleotide is of the same orientation as SEQ ID NO. 1 and is located upstream from a second oligonucleotide with respect to SEQ ID NO. 1 and wherein the second oligonucleotide is complementary to SEQ ID NO. 1, and wherein the kit may optionally comprise further components for kits known per se.

**11.** A kit according to claim 10, wherein the kit further comprises a third and a fourth oligonucleotides according to any one of claim 1 to 3, of which the third oligonucleotide is of the same orientation as SEQ ID NO. 1 and is located downstream of the first oligonucleotide, upstream of the second oligonucleotide and upstream of the fourth oligonucleotide with respect to SEQ ID NO. 1, and of which the fourth oligonucleotide is complementary to SEQ ID NO. 1.

European Patent Office

# EUROPEAN SEARCH REPORT

Application Number
EP 01 20 0057

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| X<br><br><br><br><br><br><br><br><br><br>Y | VARY P H ET AL: "USE OF HIGHLY SPECIFIC DNA PROBES AND THE POLYMERASE CHAIN REACTION TO DETECT MYCOBACTERIUM PARATUBERCULOSIS IN JOHNE'S DISEASE" JOURNAL OF CLINICAL MICROBIOLOGY,US,WASHINGTON, DC, vol. 28, no. 5, 1 May 1990 (1990-05-01), pages 933-937, XP000619662 ISSN: 0095-1137<br>* the whole document *<br>--- | 1<br><br><br><br><br><br><br><br><br><br>2-11 | C12Q1/68 |
| X<br><br>Y | WO 92 11390 A (IDEXX LAB INC) 9 July 1992 (1992-07-09)<br>* the whole document *<br>--- | 1<br><br>2-11 | |
| Y | EP 0 754 700 A (CRUACHEM LTD) 22 January 1997 (1997-01-22)<br>* the whole document *<br>--- | 1-11 | |
| Y | MILLAR DOUGLAS ET AL: "IS900 PCR to detect Mycobacterium paratuberculosis in retail supplies of whole pasteurized cows' milk in England and Wales." APPLIED AND ENVIRONMENTAL MICROBIOLOGY, vol. 62, no. 9, 1996, pages 3446-3452, XP002173058 ISSN: 0099-2240<br>* the whole document *<br>---<br>-/-- | 1-11 | TECHNICAL FIELDS SEARCHED (Int.Cl.7)<br>C12Q |

The present search report has been drawn up for all claims

| Place of search | Date of completion of the search | Examiner |
|---|---|---|
| THE HAGUE | 27 July 2001 | Hagenmaier, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number
EP 01 20 0057

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

# EUROPEAN SEARCH REPORT

Application Number
EP 01 20 0057

| Category | Citation of document with indication, where appropriate, of relevant passages | Relevant to claim | CLASSIFICATION OF THE APPLICATION (Int.Cl.7) |
|---|---|---|---|
| Y | ELLINGSON J L E ET AL: "Evaluation of the accuracy and reproducibility of a practical PCR panel assay for rapid detection and differentiation of Mycobacterium avium subspecies." MOLECULAR AND CELLULAR PROBES, vol. 14, no. 3, June 2000 (2000-06), pages 153-161, XP002173059 ISSN: 0890-8508 * the whole document * --- | 1-11 | |
| Y | FIDLER H M ET AL: "Specific detection of Mycobacterium paratuberculosis DNA associated with granulomatous tissue in Crohn's disease." GUT, vol. 35, no. 4, 1994, pages 506-510, XP001002882 ISSN: 0017-5749 * the whole document * --- | 1-11 | |
| | | | TECHNICAL FIELDS SEARCHED (Int.Cl.7) |
| Y | NEWTON: "PCR" 1994 , SPEKTRUM, AKAD. VERL. XP002173062 See page 41-page 43 --- | 1-11 | |
| Y | WO 93 09251 A (PASTEUR INSTITUT) 13 May 1993 (1993-05-13) * the whole document * --- | 1-11 | |
| A | DATABASE EMBL 'Online! ID: MPIS900A, 1989 GREEN: "Mycobacterium paratuberculosis insertion element IS900" XP002173063 * abstract * --- -/-- | | |

The present search report has been drawn up for all claims

| Place of search | Date of completion of the search | Examiner |
|---|---|---|
| THE HAGUE | 27 July 2001 | Hagenmaier, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# LACK OF UNITY OF INVENTION SHEET B

Application Number
EP 01 20 0057

The Search Division considers that the present European patent application does not comply with the requirements of unity of invention and relates to several inventions or groups of inventions, namely:

1. Claims: 1-11 (all partially)

   Invention 1:

   Oligonucleotide with Seq.ID 4 and methods for detection of M.avium ssp. paratuberkulosis using said oligonucleotide and kits comprising said oligonucleotide.

2. Claims: 1-11 (all partially)

   Inventions 2-35:

   Oligonucleotide with Seq.ID 5 and methods for detection of M.avium ssp. paratuberkulosis using said oligonucleotide and kits comprising said oligonucleotide.

   Ibidem for Seq.IDs 6-38.

European Patent Office

# EUROPEAN SEARCH REPORT

Application Number
EP 01 20 0057

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| A | DATABASE EMBL 'Online!<br>ID: MAIS901, 1991<br>DALE: "Mycobacterium avium insertion element IS901 ORF"<br>XP002173064<br>* abstract *<br>--- | | |
| A | BULL TIM J ET AL: "Characterization of IS900 loci in Mycobacterium avium subsp. paratuberculosis and development of multiplex PCR typing."<br>MICROBIOLOGY (READING),<br>vol. 146, no. 9, September 2000 (2000-09), pages 2185-2197, XP002173061<br>ISSN: 1350-0872<br>* the whole document *<br>----- | | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 27 July 2001 | Hagenmaier, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 01 20 0057

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-2001

| Patent document cited in search report | Publication date | Patent family member(s) | Publication date |
| --- | --- | --- | --- |
| WO 9211390 A | 09-07-1992 | EP 0566670 A | 27-10-1993 |
| | | US 5800984 A | 01-09-1998 |
| EP 0754700 A | 22-01-1997 | NONE | |
| WO 9309251 A | 13-05-1993 | FR 2683227 A | 07-05-1993 |
| | | AT 136592 T | 15-04-1996 |
| | | CA 2122367 A | 13-05-1993 |
| | | DE 69209846 D | 15-05-1996 |
| | | DE 69209846 T | 14-11-1996 |
| | | EP 0612356 A | 31-08-1994 |
| | | ES 2088660 T | 16-08-1996 |
| | | GR 3020265 T | 30-09-1996 |
| | | JP 7500502 T | 19-01-1995 |
| | | US 5650272 A | 22-07-1997 |

EPO FORM P0459

For more details about this annex : see Official Journal of the European Patent Office, No. 12/82

SEQUENCE LISTING

<110> Stichting Nederlands Instituut voor Zuivelonderzoe

<120> Detection of Mycobacterium paratuberculosis nuclei acids

<130> M.paratuberculosis

<140>
<141>

<160> 38

<170> PatentIn Ver. 2.1

<210> 1
<211> 1451
<212> DNA
<213> Mycobacterium paratuberculosis

<400> 1
tccttacctt tcttgaaggg tgttcggggc cgtcgcttag gcttcgaatt gcccagggac 60
gtcgggtatg gctttcatgt ggttgctgtg ttggatggcc gaaggagatt ggccgcccgc 120
ggtcccgcga cgactcgacc gctaattgag agatgcgatt ggatcgctgt gtaaggacac 180
gtcggcgtgg tcgtctgctg ggttgatctg gacaatgacg gttacggagg tggttgtggc 240
acaacctgtc tgggcgggcg tggacgccgg taaggccgac cattactgca tggttattaa 300
cgacgacgcg cagcgattgc tctcgcagcg ggtggccaac gacgaggccg cgctgctgga 360
gttgattgcg gcggtgacga cgttggccga tggaggcgag gtcacgtggg cgatcgacct 420
caacgccggc ggcgccgcgt tgctgatcgc cttgctcatc gctgccgggc agcggctgct 480
ttatattccc gggcgcacgg tccatcacgc cgcgggtagt taccgcggcg aaggcaagac 540
cgacgccaaa gacgctgcga tcatcgccga tcaggcccgg atgcgccacg acttgcagcc 600
tctgcgcgcc ggcgatgaca tcgcagtcga gctgcgcatc ctgaccagcc gacgttccga 660
tctggtggct gatcggaccc gggcgatcga accgaatgcg cgcccagctg ctggaatact 720
ttcggcgctg gaacgcgcct tcgactacaa caagagccgt gccgcgctga tcctgcttac 780
tggctaccaa actcccgacg cgctgcgcag cgccggtggc gctcgagtag ccgcgttctt 840
gcgtaaacgc aaggcccgca acgccgatac cgtcgcagcc accgcgctgc aggccgctaa 900
cgcccaacac agcatcgtgc ccggccaaca actggcggcc actgtggtgg cccgcctggc 960
caaggaggtg atggccctcg acaccgaaat cggcgacacc gacgcgatga tcgaggagcg 1020
atttcgccgc caccgccacg ccgaaatcat cctgagcatg cccggattcg gcgtcatcct 1080
gggcgctgag ttcctcgccg ccaccggcgg ggacatggcc gcattcgcct ccgccgaccg 1140
cctcgccggc gtcgccggcc tggcgccggt accacgagat tccggccgca tcagcggaaa 1200
cctcaaacgc ccccgacgct acgaccggcg cctgctgcgc gcctgctacc tgtcggcctt 1260
ggtcagcatc cgcaccgacc cctcctcgcg cacctactac gaccgaaaac gcaccgaagg 1320
aaaacgccac acccaagccg tcctcgccct ggcccgccgc cgcctcaacg tcctgtgggc 1380
catgctgcgc gaccacgctg tctaccaccc cgcaaccact accgcggcgg cttgacaacg 1440
tcattgagaa t 1451

<210> 2
<211> 1472
<212> DNA
<213> Mycobacterium avium

<400> 2
ttccttaggg ttgaaggggt ctgggattgg atgtcctgga tgcccaggac ggtggggtgt 60
ggctaatgtg ttgttgcaac ggttgttgct tgaaaggaat ggccgccctg ccgttttgga 120
cggtcctggc cactgattga gatctgacgc gtactcgatg acgctgctct aaggacctgt 180
tggcggggtt gtccgcgggg actgcgacga caggagtagc ggtatggccg aacccgaccg 240
agtgtgggtg ggtatcgacg tcggtaagtc cactcatcat gcgtgcgcga tcgatgacac 300

```
cggaaaggtg gtgtggtcga agaaaatccc gaacgaacag gccgcgatcg aagacctgat 360
cgcccagggc ggccggattg ctaaccacgt ggtgtgggcg atcgatttga cctcgcgccg 420
gcggcggctg ctgatcgccg tactgctgag cgcgaaagcc gaggtggtgt atgtgccggg 480
ccgcacggtt aacacgatga gtcatgcgtt ccgcggcgaa ggcaagaccg acgccaaaga 540
cgcgcgggta atcgccgaaa ccgctcggca ccgacgagat ctgtccccgg tcgtacccgg 600
cgaagacctg gttgccgaat tgcggtcgct gaccgcatac cggtcggatc tgatggctga 660
ctgggtgcga ggcgtgaacc gcgtgcgctc gatgctcacc gccatcttcc ctgctctgga 720
agctgcgttc gactactcca cccgcgcgcc gttgatcctg gtatccgcta tgtgcactcc 780
gggcgaaatc cggtcggcaa aaagagctgg cgtgatcaag caccttcgga aaaaccgggc 840
atggcccaac aacatcgaca cgatcgccga caagggcctc gccgcggcag caggccagat 900
aatcaccctt cccggcgaag ccggaaccgc cgcgctcatc aagcaactcg cagcacggct 960
gctggacttg gatcggcaga tcaaggacat cgataagcaa atcaccaaca aatttcgtga 1020
gcatcccagc gccgccatca tcgagtcgat gcccggcatg ggccacacc tgggcgctga 1080
gttcctcgta atcaccggcg gcaacatggc cgccttcacc aaccccggcc gactggcatc 1140
gttcgccgga ttggtacccg tcccacgcga ttccggccgt atcaccggca atctgcatcg 1200
gcccaagcgc tacaaccggc gcctgcgccg cgtgttctac ctcgccgccc tgtccagcct 1260
caagatcgaa ggtccctcgc gggctttcta cgaccgcaaa cgatccgaga accatatcca 1320
cacccaggcc ctgcttgccc tggcacgccg ccacgtcgac gtcctgtggg cactgctgcg 1380
cgacaacaga acatggcaac cccagcaacc aaccgtggca gctgcctgac gcactcaccg 1440
ggcgtcctca ccgcttgaca cgctcattga ga                               1472
```

<210> 3
<211> 1554
<212> DNA
<213> Mycobacterium avium

<400> 3

```
cgatcatgct gtccttacct ttcttgcagg gtggtcgtgg ccgagggcct taggcttcga 60
actgcccagg gacgtcgggt atggctttca tgcgtttgcc gtctgtgatg gccgaaggag 120
attggccgcc cagcgtcctg cgacgactcg accgctaatt gagagatgcg atttgatcgc 180
tgtgtaagga cacgtcggcg tggtcgtctg ctgggttgat gatgacgata atgaccatgg 240
aggtggttgt gacgcaacaa ctgtgggctg gcgttgatgc cggaaagtcc gaccaccatt 300
gcgtggtcat cgatgcagag ggcaaccgac tgctttctca gcggatcccc aacgatgaga 360
gttcgctgct gcaagtgatc caggcggtca cgaggctggc cgatggcggc gaggtcacct 420
gggcggtcga cctcaaccac ggcggggccg cgctgctgat caccctgctc atcactgcag 480
accagcggct gctctatatc cctggccgca ccgtctacca cgcttctggc gggtatcgcg 540
gcgatggaaa gaccgacgcc aaagacgctg cggtgatcgc cgatcaggcc cggatgcgcc 600
gcgatctgca accactgcgc cctggcgacg agatcgccgt ggacctgcgc atcctgaccg 660
cccgacgcag cgatctggtc gccgatcgca cccgggcgat caaccggctg cgcgcccagc 720
tgctggaata cttccccgcc ctggaacgtg cctttgacta cagctccagc aaggccgcgc 780
tgatcctgct caccgggtac caaactcccg atggattgcg ccgtgccggg gtcacccgac 840
ttgcggcctg gctgcgtaaa cgcaaggccc gcaatgctga tggtgtggca gctgcggcga 900
tcgaggcggc caacgcccaa cacacgatag ttcccggcca acaactggcc gccgcgatgg 960
tgggccggct ggctaaggag gtgatgaccc tcgacaccga aatcggcgcc accgacgtga 1020
tgatcgagga ccgatttcgc cgccaccgcc acgccgagat catcctgagc atgcccggct 1080
tcggcgtcat actcggcgct gaatttctcg ccgccaccgg aggcgacatg accgcctttg 1140
acagcgtcga tcgccttgcc ggcgtctccg ggctggcccc ggtaccgcga gactccggac 1200
gcatcagcgg caacctcaaa cgcccccgcc gctacgaccg acgactgctg cgcacctgct 1260
acctgtcggc ccaaatcgcc atccgcaccg acccggcttc gcgcacctac tacgaccgca 1320
aacgcgctga aggcaaaacc catacccaag ccgtcctcgc cctggcccgc cgacgcctca 1380
acgtcttgtg ggccatgctg cgcgaccacg ctgtctacca ccccgcaacc actactgcgg 1440
ctgcttgaca acgtcattga gaatctcctt cgatggctgc tgcggtggcc aatggttcac 1500
cggatgctcg agagtggtcc aaaggtattg cgtggcatag gaacgccagg gccg       1554
```

<210> 4
<211> 23
<212> DNA
<213> Artificial Sequence

<220>
<223> Description of Artificial Sequence:
M.paratuberculosis IS900-based sense primers

<400> 4
cttacctttc ttgaagggtg ttc 23

<210> 5
<211> 22
<212> DNA
<213> Artificial Sequence

<220>
<223> Description of Artificial Sequence:
M.paratuberculosis IS900-based sense primers

<400> 5
gtatggcttt catgtggttg ct 22

<210> 6
<211> 22
<212> DNA
<213> Artificial Sequence

<220>
<223> Description of Artificial Sequence:
M.paratuberculosis IS900-based sense primers

<400> 6
acctttcttg aagggtgttc gg 22

<210> 7
<211> 22
<212> DNA
<213> Artificial Sequence

<220>
<223> Description of Artificial Sequence:
M.paratuberculosis IS900-based sense primers

<400> 7
cctttcttga agggtgttcg gg 22

<210> 8
<211> 23
<212> DNA
<213> Artificial Sequence

<220>
<223> Description of Artificial Sequence:
M.paratuberculosis IS900-based sense primers

<400> 8
ggctttcatg tggttgctgt gtt 23

<210> 9

<211> 23
<212> DNA
<213> Artificial Sequence

<220>
<223> Description of Artificial Sequence:
M.paratuberculosis IS900-based sense primers

<400> 9
gctttcatgt ggttgctgtg ttg 23

<210> 10
<211> 23
<212> DNA
<213> Artificial Sequence

<220>
<223> Description of Artificial Sequence:
M.paratuberculosis IS900-based sense primers

<400> 10
ctttcatgtg gttgctgtgt tgg 23

<210> 11
<211> 21
<212> DNA
<213> Artificial Sequence

<220>
<223> Description of Artificial Sequence:
M.paratuberculosis IS900-based sense primers

<400> 11
gtcgtctgct gggttgatct g 21

<210> 12
<211> 21
<212> DNA
<213> Artificial Sequence

<220>
<223> Description of Artificial Sequence:
M.paratuberculosis IS900-based sense primers

<400> 12
tcgtctgctg ggttgatctg g 21

<210> 13
<211> 24
<212> DNA
<213> Artificial Sequence

<220>
<223> Description of Artificial Sequence:
M.paratuberculosis IS900-based sense primers

<400> 13

ttgatctgga caatgacggt tacg 24

<210> 14
<211> 21
<212> DNA
<213> Artificial Sequence

<220>
<223> Description of Artificial Sequence:
M.paratuberculosis IS900-based sense primers

<400> 14
aggtggttgt ggcacaacct g 21

<210> 15
<211> 21
<212> DNA
<213> Artificial Sequence

<220>
<223> Description of Artificial Sequence:
M.paratuberculosis IS900-based sense primers

<400> 15
gtggttgtgg cacaacctgt c 21

<210> 16
<211> 22
<212> DNA
<213> Artificial Sequence

<220>
<223> Description of Artificial Sequence:
M.paratuberculosis IS900-based sense primers

<400> 16
atggttatta acgacgacga gc 22

<210> 17
<211> 22
<212> DNA
<213> Artificial Sequence

<220>
<223> Description of Artificial Sequence:
M.paratuberculosis IS900-based sense primers

<400> 17
ggttattaac gacgacgcgc ag 22

<210> 18
<211> 20
<212> DNA
<213> Artificial Sequence

<220>

<223> Description of Artificial Sequence:
M.paratuberculosis IS900-based sense primers

<400> 18
cgcgctgctg gagttgattg 20

<210> 19
<211> 20
<212> DNA
<213> Artificial Sequence

<220>
<223> Description of Artificial Sequence:
M.paratuberculosis IS900-based sense primers

<400> 19
gcgctgctgg agttgattgc 20

<210> 20
<211> 18
<212> DNA
<213> Artificial Sequence

<220>
<223> Description of Artificial Sequence:
M.paratuberculosis IS900-based sense primers

<400> 20
ggcgatcgac ctcaacgc 18

<210> 21
<211> 19
<212> DNA
<213> Artificial Sequence

<220>
<223> Description of Artificial Sequence:
M.paratuberculosis IS900-based sense primers

<400> 21
cgccaaagac gctgcgatc 19

<210> 22
<211> 20
<212> DNA
<213> Artificial Sequence

<220>
<223> Description of Artificial Sequence:
M.paratuberculosis IS900-based sense primers

<400> 22
gagctgcgca tcctgaccag 20

<210> 23
<211> 20

<212> DNA
<213> Artificial Sequence

<220>
<223> Description of Artificial Sequence:
M.paratuberculosis IS900-based sense primers

<400> 23
atcctgacca gccgacgttc 20

<210> 24
<211> 24
<212> DNA
<213> Artificial Sequence

<220>
<223> Description of Artificial Sequence:
M.paratuberculosis IS900-based anti-sense primers

<400> 24
gtcgttaata accatgcagt aatg 24

<210> 25
<211> 23
<212> DNA
<213> Artificial Sequence

<220>
<223> Description of Artificial Sequence:
M.paratuberculosis IS900-based anti-sense primers

<400> 25
taaccgtcat tgtccagatc aac 23

<210> 26
<211> 22
<212> DNA
<213> Artificial Sequence

<220>
<223> Description of Artificial Sequence:
M.paratuberculosis IS900-based anti-sense primers

<400> 26
ctccgtaacc gtcattgtcc ag 22

<210> 27
<211> 22
<212> DNA
<213> Artificial Sequence

<220>
<223> Description of Artificial Sequence:
M.paratuberculosis IS900-based anti-sense primers

<400> 27
cctccgtaac cgtcattgtc ca 22

<210> 28
<211> 24
<212> DNA
<213> Artificial Sequence

<220>
<223> Description of Artificial Sequence:
M.paratuberculosis IS900-based anti-sense primers

<400> 28
tcgtcgttaa taaccatgca gtaa 24

<210> 29
<211> 20
<212> DNA
<213> Artificial Sequence

<220>
<223> Description of Artificial Sequence:
M.paratuberculosis IS900-based anti-sense primers

<400> 29
atcgctgcgc gtcgtcgtta 20

<210> 30
<211> 20
<212> DNA
<213> Artificial Sequence

<220>
<223> Description of Artificial Sequence:
M.paratuberculosis IS900-based anti-sense primers

<400> 30
caatcaactc cagcagcgcg 20

<210> 31
<211> 19
<212> DNA
<213> Artificial Sequence

<220>
<223> Description of Artificial Sequence:
M.paratuberculosis IS900-based anti-sense primers

<400> 31
cgtcaccgcc gcaatcaac 19

<210> 32
<211> 21
<212> DNA
<213> Artificial Sequence

<220>
<223> Description of Artificial Sequence:

M.paratuberculosis IS900-based anti-sense primers

<400> 32
gaatataaag cagccgctgc c 21

<210> 33
<211> 18
<212> DNA
<213> Artificial Sequence

<220>
<223> Description of Artificial Sequence:
M.paratuberculosis IS900-based anti-sense primers

<400> 33
ggcctgatcg gcgatgat 18

<210> 34
<211> 20
<212> DNA
<213> Artificial Sequence

<220>
<223> Description of Artificial Sequence:
M.paratuberculosis IS900-based anti-sense primers

<400> 34
gcagctcgac tgcgatgtca 20

<210> 35
<211> 20
<212> DNA
<213> Artificial Sequence

<220>
<223> Description of Artificial Sequence:
M.paratuberculosis IS900-based anti-sense primers

<400> 35
accagatcgg aacgtcggct 20

<210> 36
<211> 22
<212> DNA
<213> Artificial Sequence

<220>
<223> Description of Artificial Sequence:
M.paratuberculosis IS900-based anti-sense primers

<400> 36
gtcgggagtt tggtagccag ta 22

<210> 37
<211> 18
<212> DNA

<213> Artificial Sequence

<220>
<223> Description of Artificial Sequence:
M.paratuberculosis IS900-based anti-sense primers

<400> 37
acgcggctac tcgagcgc 18

<210> 38
<211> 18
<212> DNA
<213> Artificial Sequence

<220>
<223> Description of Artificial Sequence:
M.paratuberculosis IS900-based anti-sense primers

<400> 38
gctgcgacgg tatcggcg 18